(19) Europäisches Patentamt
European Patent Office
Office européen des brevets

(11) Publication number: **0 163 435**
**B1**

(12) # EUROPEAN PATENT SPECIFICATION

(45) Date of publication of patent specification: **26.10.88**

(51) Int. Cl.⁴: **C 07 C 67/307,** C 07 C 69/63, C 07 C 68/02, C 07 C 69/96

(21) Application number: **85303150.8**

(22) Date of filing: **02.05.85**

(54) **Preparation of 2-chloropropionic acid esters.**

(30) Priority: **23.05.84 GB 8413155**

(43) Date of publication of application:
**04.12.85 Bulletin 85/49**

(45) Publication of the grant of the patent:
**26.10.88 Bulletin 88/43**

(84) Designated Contracting States:
**AT BE CH DE FR GB IT LI LU NL SE**

(56) References cited:
**GB-A-2 051 808**
**GB-A-2 055 802**
**GB-A-2 090 835**

(73) Proprietor: **IMPERIAL CHEMICAL INDUSTRIES PLC**
**Imperial Chemical House Millbank**
**London SW1P 3JF (GB)**

(72) Inventor: **Crosby, John**
**12 Oakwood Court**
**Bowden Altrincham Cheshire (GB)**

(74) Representative: **Houghton, Malcolm John et al**
**Imperial Chemical Industries PLC**
**Legal Department: Patents**
**PO Box 6 Welwyn Garden City**
**Herts, AL7 1HD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 163 435 B1**

Courier Press, Leamington Spa, England.

**Description**

This invention relates to a process for the preparation of esters of 2-chloropropionic acid which are useful chemical intermediates in the synthesis of a variety of products including herbicidal compounds. More particularly, it relates to a process for the preparation of an alkly 2-chloropropionate of the formula $CH_3CH(Cl)COOR$, (A), in which R is alkyl, by chlorination of an alkyl lactate of the formula $CH_3CH(OH)COOR$,(B).

The compounds (A) and (B) each possess an asymmetrically substituted carbon atom and can exist in two enantiomeric forms, one of which possesses the D absolute configuration, i.e. the same absolute configuration as dextrorotory glyceraldehyde (as a standard), and the other of which possesses the L absolute configuration. These enantiomers are mirror images of each other and are optically active, rotating plane-polarized light in opposite directions. A racemic mixture of equal amounts by weight of the D— and L—enantiomers is optically inactive.

The process of the invention relates to the preparation of racemic mixtures 2-chloropropionic acid esters, to the preparation of optically active esters of high optical purity, i.e. esters in which there is a preponderance of one of the D— or L—enantiomers of 90% by weight or more.

It has long been known (Darzens, Compt.rend. [1911] *152* 1314, 1601; Gerrard and French, Nature 159 (1947), 263; Libermann, Nature *160* (1947), 903) that alkyl esters of 2-chloropropionic acid can be prepared by a process in which an alkyl lactate (B) is chlorinated using thionyl chloride to form the chlorosuphinate (C) in accordance with the equation:

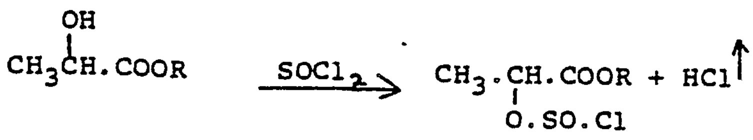

(B) (C)

and the chlorosulphinate (C) then decomposed by heating in the presence of pyridine or its hydrochloride to give the alkyl 2-chloropropionate (A) in accordance with the equation:

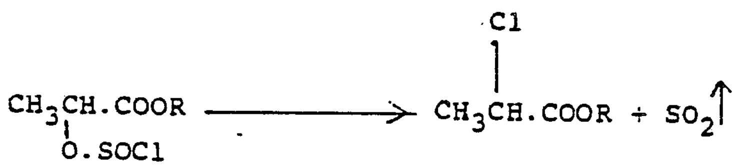

(C) (A)

Such a process has also been used for preparing optically active 2-chloropropionic acid alkyl esters from the appropriate optically active alkyl lactates (Frankland and Garner, J.Chem.Soc *105*, 1110-1115; UK Patent Specification No. 2051808).

According to Frankland and Garner, ethylsulphinyl lactate (the intermediate chlorosulphinate (C) obtained from ethyl lactate) is very stable even at 120°C and pyridine or another suitable base is employed to catalyse decomposition at a lower temperature. Frankland and Garner point out, however, that prolonged treatment with pyridine hydrochloride can cause partial racemisation of the optically active chloropropionate. In UK 2051808 it is said that where it is desired to avoid partial racemisation the amount of pyridine used should be kept within the range of from 0.05% to 2%, preferably from 0.1% to 1%, by weight of lactate. Further, a temperature of at least 60°C is required to decompose the chlorosulphinate intermediate and the temperature must not exceed 80°C if partial racemisation caused by operating at too high a temperature is to be avoided as far as possible.

The present invention provides a more robust process for the preparation of optically active chloropropionic acid esters in that the process is not sensitive in the same way to pyridine usage and temperatures lower than 60°C can be used for formation of the final product.

It is also known to react ethyl lactate and its L-enantiomer with phosgene to form the chloroformate, $CH_3CH(OCOCl)CO_2Et$, which is then used for the preparation of certain peptide carbazates for the treatment of pancreatitis (Chem.Abs. *88*p. 7374a) or carbamate esters of physiologically active phenethylamines (Chem.Abs *79*p. 73433u).

According to one aspect of the present invention there is provided a process for the preparation of 2-chloropropionic acid esters of the formula (I):

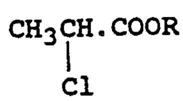

(I)

in which R is $C_{1-6}$ alkyl having a straight or branched carbon chain, preferably $C_{1-4}$ alkyl, which comprises decomposing a compound of the formula (II):

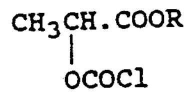 (II)

in the presence of a tertiary base.

By the term "tertiary base" is meant an organic compound containing a group

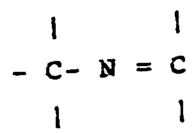 or 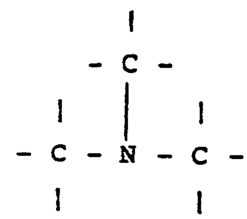 .

and which is capable of forming salts with acids. Such bases include tertiary amines such as trialkylamines, e.g. triethylamine, amides such as dialkyl formamides, e.g. dimethyl formamine, and aromatic tertiary bases such as dialkyl anilines and especially aromatic tertiary bases in which the nitrogen atom forms part of a heterocyclic ring, e.g. pyridine, quinoline, iso-quinoline and picolines. Sterically hindered bases, such as 2,6-lutidine, may not be satisfactory, however, and should be avoided.

Compound (II), which is the chloroformate of an alkyl lactate, may be obtained by treating an alkyl lactate of the formula (III):

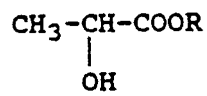 (III)

in which R has the meaning given below, with phosgene preferably, as later described, in the presence of a tertiary base. The chloroformate may be the D— or L—enantiomer or any mixture thereof, including a racemic mixture, obtained from the appropriate enantiomer or enantiomeric mixture of the alkyl lactate.

The invention also includes the D— and L—enantiomers of the chloroformate compounds (II), other than the known ethyl ester, i.e. compound (II) in which R is ethyl, and mixtures thereof including a racemic mixture. In particular it includes the D— and L—enantiomers of the compound (IV):

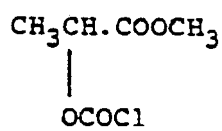 (IV)

and mixtures thereof including a racemic mixture.

In another aspect of the invention there is provided a process for the preparation of 2-chloropropionic acid esters (I) which comprises the steps of (a) reacting an alkyl lactate (III) with phosgene and (b) decomposing the reaction product so formed, each step being carried out in the presence of a tertiary base. The tertiary bases used in steps (a) and (b) may be the same or different.

Preferably step (b) is carried out in an inert organic solvent, additional to the tertiary base, to prevent solidification of the reaction mass. As step (b) can be carried out at a temperature below 60°C, the solvent used may be relatively low boiling to facilitate later recovery and re-use. It may also be used to advantage in step (a) of the process in which case it is one which is inert to phosgene and in which phosgene is soluble. Dichloromethane is situable but other higher boiling chlorinated hydrocarbons, e.g. chlorobenzene, could be used. Where the process is to be used for the preparation of an optically active chloropropionate, step (b) is usually carried out at a temperature in the range of 20 to 40°C, ideally 25 to 35°C, to avoid racemisation. Also, the solvent should be one which does not effect the stereochemistry of the reaction.

In step (a) of the process, reaction between phosgene and the alkyl lactate would be very slow without, at least, a catalytic amount of tertiary base present. If a catalytic amount of tertiary base is used, it is desirable to have another acid binder present to remove hydrogen chloride formed during reaction.

To ensure complete reaction between the phosgene and alkyl lactate, it is preferable, however, to use at least 1 mol of tertiary base for each mol of lactate. It is also desirable that for each mol of tertiary base present there should be used more than 1 mol, preferably at least 1.3 mol and more preferably at least 2 mol, typically 2.1 mol, of phosgene. Without prejudice to the generality of the invention, it is thought that the tertiary base forms a complex with the phosgene and that the complex is the reactive species. For

example, if pyridine is used as the tertiary base, it is believed that pyridine complexes with the phosgene according to the equation:

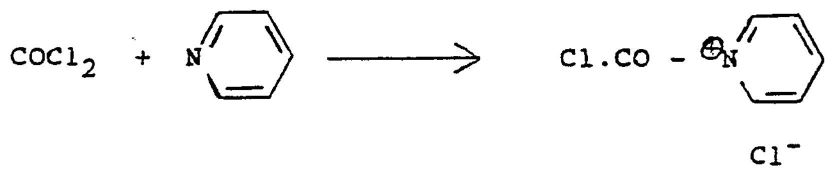

(V)

and that the pyridine/phosgene complex (V) then reacts with the alkyl lactate (III) as follows:

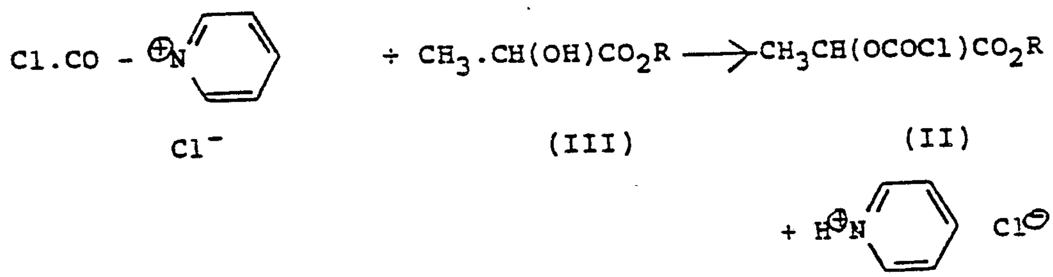

(III) (II)

If excess free tertiary base is present at step (a), there is evidence that this leads to the formation of unwanted by-products, possibly including a condensation production of the formula

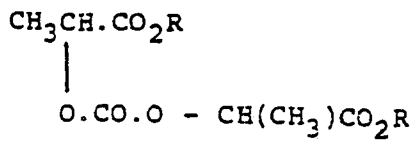

This side reaction can largely be avoided by (i) having sufficient tertiary base present to ensure complete reaction of the alkyl lactate with the phosgene, and (ii) having sufficient phosgene present to eliminate free tertiary base.

In carrying out step (a), it is desirable, therefore, to mixt the tertiary base and phosgene before contact with the alkyl lactate. Conveniently, the lactate is added dropwise to the mixture of phosgene and pyridine, preferably suspended in a solvent for the lactate, at a temperature in the range of from −5 to 10°C. The amount of solvent used is not critical but typically there will be sufficient to give a 10 to 30% w/v solution of lactate.

In a preparation in which 1 mol of lactate is used, the lactate can be added to the pyridine/phosgene suspension over about 2 hours to maintain the temperature at 0 to 5°C and cause only gentle refluxing of the phosgene. The reaction mixture is then stirred for, say, another 4 hours while allowing the temperature to rise to 20 to 25°C and then excess phosgene removed by, for example, purging the reaction mixture with nitrogen for several hours.

Alternatively, it may be more expedient to distil off the phosgene and low-boiling solvent, such as dichloromethane, where one is used, if necessary under reduced pressure, and recover the phosgene/solvent distillate for re-use. A distillation temperature of up to about 40°C should be acceptable without risk of racemisation of an optically active chloropropionate, if this is being prepared.

In progressing to step (b) of the process, the temperature of the de-gassed reaction mixture is preferably adjusted to about 20°C and solvent or additional solvent added, as necessary. Further tertiary base is added — about 0.2 mol per mol lactate has been found adequate but more may be used if desired — and the reaction mixture stirred at a maintained temperature of from 20 to 40°C, preferably 25 to 35°C, until the decomposition of the chloroformate intermediate (II) to the chloropropionate (I) is complete, i.e. when evolution of carbon dioxide gas ceases.

The chloropropionate (I) may be isolated by conventional methods, for example, by washing the organic reaction mixture with dilute acid, e.g. dilute hydrochloric acid; to remove tertiary base, and then with water to remove acid; drying the organic layer over, for example, magnesium sulphate; and distilling off the solvent at a temperature of 40°C or below, if necessary under reduced pressure. The product may then be purified by distillation under reduced pressure at a temperature preferably not exceeding 40°C.

It will be appreciated that the foregoing instructions to maintain processing temperatures at below 40°C are aimed at the preparation of products of high optical purity. Higher temperatures may be used where the risk of racemisatioin can be tolerated or where racemic products are being prepared.

The process according to the invention can be used for the preparation of racemic alkyl 2-chloropropionates from racemic alkyl lactates. The racemic alkyl 2-chloropropionates thus obtained can be used, after saponification if required, in the synthesis of certain 2-chloropropionic acid derivatives, including 2-[4-(5-trifluoromethyl-2-pyridyloxy)phenoxy]propionic acid, esters thereof and related products, known for their herbicidal properties.

The process is of especial value for the preparation of optically active alkyl 2-chloropropionates, which predominantly or totally comprise one of the optical isomers, from alkyl lactates which are themselves optically active, the conversion of the alkyl lactate to the alkyl 2-chloropropionate taking place with an inversion of configuration of the Walden inversion type. In particular, the process may be used to prepare alkyl L-2-chloropropionates from the corresponding alkyl D-lactates and alkyl D-2-chloropropionates from the corresponding alkyl L-lactates. The alkyl L-2-chloropropionates obtained according to the process of the invention can be used in the synthesis of D-2-phenoxypropionic acids,in particular D-2-[4-(5-trifluoromethyl-2-pyridyloxy)phenoxy] propionic acid and esters thereof, which are herbicidally more active than the corresponding racemic products.

The invention is illustrated by the following Examples in which percentages are by weight and the following abbreviations are used:

| | |
|---|---|
| °C | degrees centigrade |
| ml | millilitres |
| g | grams |
| c | concentration, g/100ml |
| MW | molecular weight |
| $[a]^{30}$ | Specific rotation of plane polarised |
| D | sodium light (the D line) at 30°C |
| + | dextroration |
| − | laevorotation |
| nmr | nuclear magnetic resonance |
| enantiomeric excess | percentage excess by weight of one enantiomer over another [e.g. in a mixture of 97% by weight of a D-isomer and 3% by weight of an L-isomer, the D-isomer is present in 94% (97−3%) enantiomeric excess] |
| $CCl_4$ | carbon tetrachloride |
| HCl | Hydrochloric acid |
| b.pt | boiling point |
| mmHg | pressure measured in millimetres of mercury |
| $cm^{-1}$ | per centimetre |
| $1.hr^{-1}$ | litres per hour |

EXAMPLE 1

Preparation of D—(+)—methyl2-chloropropionate

To pyridine (8 ml; 0.099 mol) at −5°C was added phosgene (16 ml; 0.225 mol) followed by dropwise addition of L(−) methyl lactate (0.10 mol). After allowing the mixture to warm to room temperature overnight under a purge of nitrogen, further pyridine (20 ml; 0.248 mol) and dichloromethane (80 ml) were added. The mixture was stirred at room temperature and gas was evolved. The organic solution was washed with 10% HCl and evaporated to yield 11.3g of crude D—(+)— ethyl 2-chloropropionate which after short path (Kugelrohr) distillation gave a main fraction (35% yield) of material having an optical rotation of $[a]_D^{30}$ + 31.35°C (C = 1.00, $CCl_4$) estimated by nmr to have an enantiomeric excess of at least 94%.

EXAMPLE 2

Preparation of D-(+)-methyl-2-chloropropionate

To a 1 litre glass reaction vessel, fitted with anchor agitater, thermometer, condenser and cold trap, and two dropping funnels, one also fitted with a cold trap, was charged 680g (500 ml) dichloromethane. With the apparatus vented via a caustic scrubbing system providing positive suction and fitted with a pressure/vacuum relief bubbler, the prechilled dropping funnel fitted with the cold trap was charged with 198g (2 mols, 150 ml) of liquid phosgene from a cylinder.

The reaction vessel was cooled to 0°C by means of a brine bath, following which the liquid phosgene was run into the dichloromethane as quickly as possible.

To the second dropping funnel was charged 75g (0.95 mols) of pyridine which was then slowly run into the phosgene solution in the reactor over about 2 hours, maintaining the reactor temperature at 0 to 5°C and minimising refluxing in the cold trap/condenser. A pale cream solid (pyridine-phosgene complex) was precipitated. After addition of the pyridine, the dropping funnel was charged with 104g (1 mol) L-methyl-lactate, which was then run into the reactor over about 2 hours at a rate which maintained the temperature at 0 to 5°C and caused only gentle refluxing of the phosgene. The reactor contents were then stirred for 4 hours, during which time the temperature rose to 20 to 25°C. A white slurry, believed to be the intermediate chloroformate derivative with pyridine hydrochloride, was obtained. This was de-gassed by passing a stream of nitrogen through the reactor for 36 hours to remove excess phosgene which was absorbed in the scrubber system. Some carry-over of dichloromethane also occurred. The reactor temperature rose to 30°C.

To the resulting slurry, cooled to 20°C, was charged 15.8g (0.2 mol) pyridine over about 20 minutes, the temperature being maintained at 25 to 30°C. After completion of the addition (during which a red colouration occurred; the reaction mass was stirred for 24 hours. 36% Hydrochloric acid (25g) in water

(80g) was then added and the reaction mass stirred for a further 30 minutes before the agitation was stopped and the mass allowed to settle into two layers. The lower organic layer was transferred to a separating funnel, washed with 3 × 250g water and dried over magnesium sulphate. After screening off the drying agent, it was distilled under partial pressure at 30-35°C, to remove dichloromethane. The crude, reddish brown coloured product remaining weighed 110.7g, and darkened on standing. It was distilled under water pump vacuum at a maximum temperature of 40°C. A fores fraction (1.5g) and a main fraction (96.0g) were collected leaving a still residue of 3.5g. Some dichloromethane was initially distilled off before the fores fraction. The main fraction (96.0g) (78.3% theory; NW 122.5), gave an optical rotation $[\alpha]_D^{30}$ + 32.15° : D:L radio (90:2); (96% enantiomeric excess).

EXAMPLE 3

Preparation of (L)-methyl 2-(chlorocarbonyloxy)propionate[(L)—$CH_3CH)O-CO-Cl)CO_2CH_3$

Following the procedure of the first of Example 2, dichloromethane (500 ml) was charged to the reactor which was cooled to 0°C. Phosgene (145 ml, 198 g) was condensed and added to the chilled dichloromethane. Pyridine (79 g) was then run into the phosgene solution whilst keeping the temperature at 0 to 5°C. (L)-Methyl lactate (104 g) was then added, again keeping the temperature at 0 to 5°C. The temperature was allowed to rise to ambient and the reaction mass stirred for 20 hours, the temperature being raised to 30°C during the latter part of the stirring period. The mixture was then degassed with nitrogen to remove excess phosgene. A solution of concentrated HCl (25 g) in water (80 ml) was added and the resulting two phase mixture stirred for $\frac{1}{4}$ to $\frac{1}{2}$ hour. The lower organic layer was separated, dried over magnesium sulphate, and distilled under reduced pressure to remove the dichloromethane.

The crude product was distilled to give a main fraction (125.8 g) with b.pt 104°C at 6l mmHg and $[\alpha]_D^{30}$ −21.81· (c=1, $CCl_4$).

EXAMPLES 4 AND 5

Preparation of (L)-*iso*propyl 2-(chlorocarbonyloxy) propionate [(L)-$CH_3CH(OCOCl)CO_2CH(CH_3)_2$] and (L)-butyl 2-(chlorocarbonyloxy) propionate [(L)-$CH_3CH(OCOCl)CO_2C_4H_9$]

These compounds were prepared in similar manner using the procedure for Example 3 except that (L)-*iso*propyl lactate and (L)-butyl lactate were used in place of (L)-methyllactate. They were characterised as follows:

| Example No. | Formula | b.pt | $[\alpha]^{30}_{D}$ | $(CCl_4)$ c |
| --- | --- | --- | --- | --- |
| 4 | (L)-$CH_3CH(OCOCl)CO_2CH(CH_3)_2$ | 112°C at 52 mmHg | -18.70° | 0.87 |
| 5 | (L)-$CH_3CH(OCOCl)CO_2C_4H_9$) | 126°C at 44 mmHg | -11.72° | 1.3 |

The chloroformates of Examples 3, 4 and 5 all showed strong infra-red absorptions at 1750-1760 and 1780-1785 $cm^{-1}$.

## EXAMPLE 6

### Decomposition of (L)-methyl 2-(chlorocarbonyloxy)propionate to give (D)-methyl 2-chloropropionate

The (L)-methyl 2-(chlorocarbonyloxy)propionate prepared in Example 3 (40 g) and dichloromethane (160 g) were charged to a 250 ml conical flask fitted with a thermometer and connected to a gas meter. The solution was agitated magnetically at 30°C and pyridine (5.3 g) was added quickly. The temperature rose to 37°C, stayed at 37°C for 5 minutes and was then restored to 30°C. Carbon dioxide gas evolution, which reached a maximum of 12 1 $hour^{-1}$, ceased after two hours at which point the solution was acidified. (5 ml 36% HCl in 30 ml water) and washed with water (3 × 50 ml). After drying over magnesium sulphate the dichloromethane was evaporated under reduced pressure. The crude product was distilled to give a main fraction (12.5 g); b.pt 52°C at 40 mmHg; $[\alpha]_D^{30}$ + 29.9° (c=0.99,$CCl_4$); infra red: 1750 $cm^{-1}$.

## EXAMPLES 7 TO 10

### Decomposition of (L)-methyl, (L)-*iso*propyl and (L)-butyl esters of 2-(chlorocarbonyloxy)propionic acid.

These compounds were decomposed to give the (D)-methyl, (D)-*iso*propyl and (D)-butyl esters of 2-chloropropionate by the procedure described in Example 6 except that triethylamine and 4-picoline were used as decomposition catalysts in place of pyridine in Examples 7 and 8. Results and other procedural variations are recorded in Table 1 below.

TABLE 1

| Example No. | Chloroformate ester | Source of ester | Rotation of distilled chloroformate | Rotation of distilled 2-chloro-propionate | Base | Mol ratio Base: chloro-formate | Decomp Temp (°C) | Decomp Time (hrs) | Max $CO_2$ evaluation $l. hr^{-1}$ |
|---|---|---|---|---|---|---|---|---|---|
| 7 | Methyl | Example 3 | -21.81° | +27.77° | triethyl-amine | .0.2 | Exotherm to 40-45°C Cooled to 50°C over 15 min | 0.50 | Too fast to measure |
| 8 | Methyl | Repeat of Example 3 | -21.78° | +32.50° | 4-picoline | 0.2 | 35°C | 6.75 | 2.0 |
| 9 | *iso*-propyl | Example 4 | -18.70° | +16.54° | pyridine | 0.2 | 30°C | 2.20 | 6.0 |
| 10 | *n*-butyl | Example 5 | -11.72° | +12.78° | pyridine | 0.2 | 30°C | 2.00 | 7.0 |

MJH/jlw
PP 33129
26 April 1985

**Claims for the Contracting States: BE CH DE FR GB IT LI LU NL SE**

1. A process for the preparation of 2-chloropropionic acid esters of the formula (I):

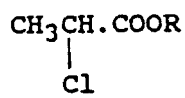

(I)

in which R is $C_{1-6}$ alkyl having a straight or branched carbon chain, which comprises decomposing a compound of the formula (II):

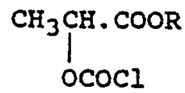

(II)

in the presence of a tertiary base.

2. A process for the preparation of 2-chloropropionic acid esters of the formula (I):

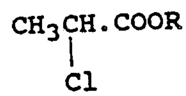

(I)

in which R is $C_{1-6}$ alkyl having a straight or branched carbon claim, which comprises the steps of (a) reacting an alkyl lactate of the formula (III):

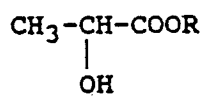

(III)

with phosgene and (b) decomposing the reaction product so formed, each of steps (a) and (b) being carried out in the presence of a tertiary base.

3. A process according to claim 1 or 2 in which decomposition is carried out in an inert organic solvent.

4. A process according to any one of the preceding claims in which decomposition is carried out at a temperature below 60°C.

5. A process according to any one of the preceding claims for the preparation of an optically active ester (I) in which decomposition is carried out at a temperature in the range of 20 to 40°C.

6. A process according to claim 2 in which in step (a) at least one mole of tertiary base is used for each mol of lactate (III).

7. A process according to claim 2 or 6 in which there is used more than 1 mol of phosgene for each mol of tertiary base used in step (a).

8. D- and L-enantiomers of chloroformate compounds of the formula (II)

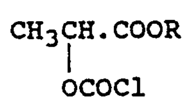

(II)

in which R is $C_1$ or $C_{3-6}$ alkyl and mixtures thereof, including racemic mixtures.

9. The compounds (L)-methyl 2-(chlorocarbonyloxy)propionate, (L)-isopropyl 2-(chlorocarbonyloxy)-propionate, (L)-butyl 2-(chlorocarbonyloxy)propionate, their D-enantiomers and mixtures of the D- and L-enantiomers, including racemic mixtures.

**Claims for the Contracting States: AT**

1. A process for the preparation of 2-chloropropionic acid esters of the formula (I):

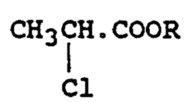

(I)

in which R is $C_{1-6}$ alkyl having a straight or branched carbon chain, which comprises decomposing a compound of the formula (II):

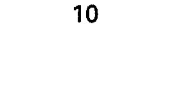

(II)

in the presence of a tertiary base.

2. A process for the preparation of 2-chloropropionic acid esters of the formula (I)

$$\underset{\displaystyle Cl}{CH_3CH}.COOR \qquad (I)$$

in which R is $C_{1-6}$ alkyl having a straight or branched carbon claim, which comprises the steps of (a) reacting an alkyl lactate of the formula (III)

$$CH_3-\underset{\displaystyle OH}{CH}-COOR \qquad (III)$$

with phosgene and (b) decomposing the reaction product so formed, each of steps (a) and (b) being carried out in the presence of a tertiary base.

3. A process according to claim 1 or 2 in which decomposition is carried out in an inert organic solvent.

4. A process according to any one of the preceding claims in which decomposition is carried out at a temperature below 60°C.

5. A process according to any one of the preceding claims for the preparation of an optically active ester (I) in which decomposition is carried out at a temperature in the range of 20 to 40°C.

6. A process according to claim 2 in which in step (a) at least one mole of tertiary base is used for each mol of lactate (III).

7. A process according to claim 2 or 6 in which there is used more than 1 mol of phosgene for each mol of tertiary base used in step (a).

**Patentansprüche für die Vertragsstaaten: BE CH DE FR GB IT LI LU NL SE**

1. Verfahren zur Herstellung von 2-Chlorpropionsäureestern der Formel I:

$$\underset{\displaystyle Cl}{CH_3CH}.COOR \qquad (I)$$

worin R für $C_{1-6}$-Alkyl mit gerader oder verzweigter Kohlenstoffkette steht, bei welchem eine Verbindung der Formel II

$$\underset{\displaystyle OCOCl}{CH_3CH}.COOR \qquad (II)$$

in Gegenwart einer tertiären Base zersetzt wird.

2. Verfahren zur Herstellung von 2-Chlorpropionsäureestern der Formel I

$$\underset{\displaystyle Cl}{CH_3CH}.COOR \qquad (I)$$

worin R für $C_{1-6}$-Alkyl mit gerader oder verzweigter Kohlenstoffkette steht, bei welchem (a) ein Alkyllactat der Formel III

$$CH_3-\underset{\displaystyle OH}{CH}-COOR \qquad (III)$$

mit Phosgen umgesetzt wird und (b) das so gebildete Reaktionsprodukt zersetzt wird, wobei die Stufen (a) und (b) in Gegenwart einer tertiären Base ausgeführt werden.

3. Verfahren nach Anspruch 1 oder 2, bei welchem die Zersetzung in einem inerten organischen Lösungsmittel ausgeführt wird.

4. Verfahren nach einem der vorhergehenden Ansprüche, bei welchem die Zersetzung bei einer Temperatur unter 60°C ausgeführt wird.

5. Verfahren nach einem der vorhergehenden Ansprüche zur Herstellung eines optisch aktiven Esters (I), bei welchem die Zersetzung bei einer Temperatur im Bereich von 20 bis 40°C ausgeführt wird.

6. Verfahren nach Anspruch 2, bei welchem in der Stufe (a) mindestens 1 Mol tertiäre Base für jedes Mol Lactat (III) verwendet wird.

7. Verfahren nach einem der Ansprüche 2 oder 6, bei welchem mehr als 1 Mol Phosgen für jedes Mol in der Stufe (a) verwendeter tertiärer Base zur Verwendung gelangt.

8. D- und L-Enantiomere von Chlorformiatverbindungen der Formel (II)

$$\underset{\displaystyle OCOCl}{CH_3\underset{|}{C}H.COOR} \qquad (II)$$

worin R für $C_1$ oder $C_{3-6}$-Alkyl und Gemischen davon steht, einschließlich racemischer Gemische.

9. Die Verbindungen (L)-Methyl-2-(chlorcarbonyloxy)propionat, (L)-Isopropyl-2-(chlorcarbonyloxy)-propionat, (L)-Butyl-2-(chlorcarbonyloxy)propionat und deren D-Enantiomere und Gemische aus den D- und L-Enantiomeren, einschließlich racemischer Gemische.

**Patentansprüche für den Vertragsstaat: AT**

1. Verfahren zur Herstellung von 2-Chlorpropionsäureestern der Formel I:

$$\underset{\displaystyle Cl}{CH_3\underset{|}{C}H.COOR} \qquad (I)$$

worin R für $C_{1-6}$-Alkyl mit gerader oder verzweigter Kohlenstoffkette steht, bei welchem eine Verbindung der Formel II

$$\underset{\displaystyle OCOCl}{CH_3\underset{|}{C}H.COOR} \qquad (II)$$

in Gegenwart einer tertiären Base zersetzt wird.

2. Verfahren zur Herstellung von 2-Chlorpropionsäureestern der Formel I

$$\underset{\displaystyle Cl}{CH_3\underset{|}{C}H.COOR} \qquad (I)$$

worin R für $C_{1-6}$-Alkyl mit gerader oder verzweigter Kohlenstoffkette steht, bei welchem (a) ein Alkyllactat der Formel III

$$\underset{\displaystyle OH}{CH_3-\underset{|}{C}H-COOR} \qquad (III)$$

mit Phosgen umgesetzt wird und (b) das so gebildete Reaktionsprodukt zersetzt wird, wobei die Stufen (a) und (b) in Gegenwart einer tertiären Base ausgeführt werden.

3. Verfahren nach Anspruch 1 oder 2, bei welchem die Zersetzung in einem inerten organischen Lösungsmittel ausgeführt wird.

4. Verfahren nach einem der vorhergehenden Ansprüche, bei welchem die Zersetzung bei einer Temperatur unter 60°C ausgeführt wird.

5. Verfahren nach einem der vorhergehenden Ansprüche zur Herstellung eines optisch aktiven Esters (I), bei welchem die Zersetzung bei einer Temperatur im Bereich von 20 bis 40°C ausgeführt wird.

6. Verfahren nach Anspruch 2, bei welchem in der Stufe (a) mindestens 1 Mol tertiäre Base für jedes Mol Lactat (III) verwendet wird.

7. Verfahren nach einem der Ansprüche 2 oder 6, bei welchem mehr als 1 Mol Phosgen für jedes Mole in der Stufe (a) verwendeter tertiärer Base zur Verwendung gelangt.

**Revendications pour les Etats contractants: BE CH DE FR GB IT LI LU NL SE**

1. Procédé de préparation d'esters d'acide 2-chloropropionique de formule (I):

$$\underset{\displaystyle Cl}{CH_3\underset{|}{C}H.COOR} \qquad (I)$$

dans laquelle R est un groupe alkyle en $C_1$ à $C_6$ à chaîne carbonée droite ou ramifiée, qui consiste à décomposer un composé de formule (II):

$$\begin{array}{l} CH_3CH.COOR \\ \quad\;\;| \\ \quad\;\; OCOCl \end{array} \qquad (II)$$

en présence d'une base tertiaire.

2. Procédé de préparation d'esters d'acide 2-chloropropionique de formule (I)

$$\begin{array}{l} CH_3CH.COOR \\ \quad\;\;| \\ \quad\;\; Cl \end{array} \qquad (I)$$

dans laquelle R est un groupe alkyle en $C_1$ à $C_6$ à chaîne carbonée droite ou ramifiée, qui comprend les étapes consistant (a) à faire réagir un lactate d'alkyle de formule (III)

$$\begin{array}{l} CH_3-CH-COOR \\ \quad\quad\;| \\ \quad\quad\; OH \end{array} \qquad (III)$$

avec le phosgène et (b) à décomposer le produit de réaction ainsi formé, chacune des étapes (a) et (b) étant conduite en présence d'une base tertiaire.

3. Procédé suivant la revendication 1 ou 2, dans lequel la décomposition est conduite dans un solvant organique inerte.

4. Procédé suivant l'une quelconque des revendications précédentes, dans lequel la décomposition est conduite à une température inférieure à 60°C.

5. Procédé suivant l'une quelconque des revendications précédentes, pour la préparation d'un ester (I) optiquement actif, dans lequel la décomposition est conduite à une température comprise dans l'intervalle de 20 à 40°C.

6. Procédé suivant la revendication 2, dans lequel au moins une mole de base tertiaire est utilisée pour chaque mole de lactate (III) dans l'étape (a).

7. Procédé suivant la revendication 2 ou 6, dans lequel on utilise plus l'une mole de phosgène pour chaque mole de base tertiaire utilisée dans l'étape (a).

8. Les énantiomères D et L de chloroformiates de formule (II)

$$\begin{array}{l} CH_3CH.COOR \\ \quad\;\;| \\ \quad\;\; OCOCl \end{array} \qquad (II)$$

dans laquelle R est une groupe alkyle en $C_1$ ou en $C_3$ à $C_6$ et leurs mélanges, y compris leurs mélanges racémiques.

9. Les composés: (L)-2-(chlorocarbonyloxy)propionate de méthyle, (L)-2-(chlorocarbonyloxy)-propionate d'isopropyle, (L)-2-(chlorocarbonyloxy)propionate de butyle leurs énantiomères D et des mélanges des énantiomères D et L, y compris des mélanges racémiques.

**Revendications pour l'Etat Contractant: AT**

1. Procédé de préparation d'esters d'acide 2-chloropropionique de formule (I):

$$\begin{array}{l} CH_3CH.COOR \\ \quad\;\;| \\ \quad\;\; Cl \end{array} \qquad (I)$$

dans laquelle R est une groupe alkyle en $C_3$ à $C_6$ à chaîne carbonée droite ou ramifiée, qui consiste à décomposer un composé de formule (II):

$$\begin{array}{l} CH_3CH.COOR \\ \quad\;\;| \\ \quad\;\; OCOCl \end{array} \qquad (II)$$

en présence d'une base tertiaire.

2. Procédé de préparation d'esters d'acide 2-chloropropionique de formule (I)

$$\begin{array}{l} CH_3CH.COOR \\ \quad\;\;| \\ \quad\;\; Cl \end{array} \qquad (I)$$

dans laquelle R est une groupe alkyle en $C_1$ à $C_6$ à chaîne carbonée droite ou ramifiée, qui comprend les étapes consistant (a) à faire réagir un lactate d'alkyle de formule (III)

$$CH_3-\underset{\displaystyle OH}{\underset{|}{CH}}-COOR \qquad (III)$$

avec le phosgène et (b) à décomposer le produit de réaction ainsi formé, chacune des étapes (a) et (b) étant conduite en présence d'une base tertiaire.

3. Procédé suivant la revendication 1 ou 2, dans lequel la décomposition est conduite dans un solvant organique inerte.

4. Procédé suivant l'une quelconque des revendications précédentes, dans lequel la décomposition est conduite à une température inférieure à 60°C.

5. Procédé suivant l'une quelconque des revendications précédentes, pour la préparation d'un ester (I) optiquement actif, dans lequel la décomposition est conduite à une température comprise dans l'intervalle de 20 à 40°C.

6. Procédé suivant la revendication 2, dans lequel au moins une mole de base tertiaire est utilisée pour chaque mole de lactate (III) dans l'étape (a).

7. Procédé suivant la revendication 2 ou 6, dans lequel on utilise plus d'une mole de phosgène pour chaque mole de base tertiaire utilisée dans l'étape (a).